# EUROPEAN PATENT APPLICATION

(11) **EP 3 369 447 A1**
(43) Date of publication of application: **05.09.2018**
(21) Application number: 16859887.8
(22) Date of filing: 27.10.2016
(51) Int. Cl.: A61M 5/31, A61M 5/32

(54) **GRIP AND SYRINGE ASSEMBLY**

(30) Priority: 30.10.2015 JP 2015213958
(71) Applicant: Terumo Kabushiki Kaisha, Tokyo 151-0072 (JP)
(72) Inventor: OHASHI, Hirotaka, Tokyo 163-1450 (JP); TAKEMOTO, Masafumi, Fujinomiya-shi Shizuoka 418-0004 (JP)
(74) Representative: Casalonga
(86) International application number: PCT/JP2016/081863
(87) International publication number: WO 2017/073658

(57) **Abstract**

A syringe assembly (10) is provided with a grip (16) which is attached to a syringe (12). The grip (16) is provided with: a base part (36) including a finger placement part (38); and a tubular body part (40) protruding from the base part (36) in a direction toward the leading end, wherein a fitting slit (42) is formed over the overall length of the grip (16) . The tubular body part (40) includes a small diameter section (50) and a large diameter section (52) . The large diameter section (52) is thicker than the small diameter section (50), and forms a leading end region of the grip (16).

## Description

### Technical Field

The present invention relates to a grip attached to a proximal end portion of a barrel and a syringe assembly including the grip.

### Background Art

Regarding a syringe used to administer a liquid medicine or the like to a patient, it is known that a grip including a finger rest portion protruding outward is attached to a proximal end portion of a barrel that constitutes a body portion of the syringe in order to facilitate a user to hold and operate the syringe (for example, see JP 2845426 B2). A grip called a backstop in JP 2845426 B2 includes a peripheral wall having an opening portion that opens laterally and a finger rest portion provided at a proximal end portion of the peripheral wall and is configured to be able to attach to the barrel from a side of the barrel.

When giving an injection to a patient by using a syringe where such a grip is attached to a proximal end portion of the barrel, the user holds the syringe by sandwiching the peripheral wall by, for example, the index finger and the middle finger while hooking these two fingers on the finger rest portion of the grip. Then, the user punctures a skin of the patient with a needle by pressing the syringe toward a distal end, and thereafter the user administers a liquid medicine into the body of the patient by advancing a pusher connected to a gasket.

### Summary of Invention

A conventional syringe grip disclosed in JP 2845426 B2 has a problem that the fingers easily slip toward the distal end with respect to the grip when pressing the syringe toward the distal end for needle puncture.

The present invention has been made in view of the problem of the conventional technique described above, and an object of the present invention is to provide a grip which is easily attached to a proximal end outer circumferential portion of the barrel and can effectively suppress slip of the fingers when pressing the syringe, and a syringe assembly.

To achieve the above object, the present invention provides a grip that is attached to a barrel of a syringe having a flange protruding outward at a proximal end portion of the barrel, the grip including: a base portion having a finger rest portion protruding outside of the flange and a flange mounting portion to be mounted to the flange; and a cylindrical body portion that protrudes from the base portion toward a distal end and extends in a circumferential direction so as to fit to an outer circumferential surface of the barrel, wherein a mounting slit that traverses longitudinally through a wall portion of the base portion and a wall portion of the cylindrical body portion in an axis direction is formed over an entire length of the grip, and the cylindrical body portion has a small diameter portion that is relatively thin and protrudes from the base portion toward the distal end, and a large diameter portion that extends from the small diameter portion toward the distal end to constitute a distal end region of the grip and is formed to be thicker than the small diameter portion.

According to the grip that adopts the above configuration, it is possible to easily attach the grip to the proximal end portion of the barrel through the mounting slit from the side of the barrel. Further, a constricted portion having a shape recessed inward in a radial direction is formed in an outer circumferential portion of the small diameter portion due to an outer diameter difference between the large diameter portion and the small diameter portion. Thereby, when the syringe is pressed toward the distal end in order to puncture an object to be punctured with a puncture needle provided in the syringe, it is possible to effectively prevent fingers of a user that are hooked on a constricted portion from slipping toward the distal end.

In the grip, an outer circumferential portion of the small diameter portion may have a straight portion whose outer diameter is constant over a predetermined length in the axis direction.

By this configuration, a user can more easily recognize that the user should hook fingers on the constricted portion, so that it is possible to prevent the user from hooking fingers by mistake on the outer circumferential surface of the barrel located closer to the distal end than the grip.

In the grip, a ratio of an outer diameter of the large diameter portion to an outer diameter of a thinnest portion of the small diameter portion may be 105 to 200%. In addition, in the grip, a difference between an outer diameter of the large diameter portion and an outer diameter of a thinnest portion of the small diameter portion may be 1.0 to 10 mm.

By this configuration, it is possible to form an appropriate diameter difference where the user can easily hook fingers between the large diameter portion and the small diameter portion.

In the grip, the large diameter portion may have an inner circumferential surface whose inner diameter is constant in the axis direction and which is configured to support the outer circumferential portion of the barrel.

By this configuration, it is possible to suppress rattling of the grip in a state in which the grip is attached to the barrel.

In the grip, a length of the large diameter portion along the axis direction may be 1.5 to 20 mm.

By this configuration, it is possible to suppress degradation of visibility of the liquid filled in the barrel.

In addition, the present invention provides a syringe assembly including: a syringe having a barrel; and a grip attached to a proximal end outer circumferential portion of the barrel, wherein the grip is any of the grips.

According to the syringe assembly that adopts the above configuration, when the syringe is pressed toward the distal end in order to puncture an object to be punctured with the puncture needle, it is possible to effectively prevent fingers of the user that are hooked on the constricted portion from slipping toward the distal end. Further, in a manufacturing process of the syringe assembly, it is possible to easily attach the grip to the proximal end portion of the barrel through the mounting slit from the side of the barrel.

The syringe assembly further includes a protective device attached to the syringe, wherein the syringe may have a puncture needle having a needle tip and the barrel that holds the puncture needle, the protective device may have a hollow cylindrical cover body that is relatively displaceable with respect to the barrel in an axis direction and an biasing member that biases the cover body toward a distal end with respect to the barrel, and the cover body may be configured to be relatively displaced toward a proximal end with respect to the barrel along an outer circumferential surface of the barrel by being pressed by an object to be punctured against an biasing force of the biasing member when the puncture needle is punctured into the object to be punctured, and cover the needle tip by being relatively displaced toward the distal end with respect to the barrel by the biasing force of the biasing member when the puncture needle is removed from the object to be punctured.

By the above configuration, it is possible to notify the user of the movable range of the cover body in advance before use of the syringe assembly. Therefore, when the user uses the syringe assembly, it is possible to prevent the user from hooking fingers by mistake on the outer circumferential surface of the barrel located closer to the distal end than the grip and avoid the cover body from hitting fingers of the user when the cover body moves toward the proximal end during the puncture . Thereby, it is possible to prevent blocking of the movement of the cover body toward the proximal end and avoid insufficient puncture that does not reach a prescribed depth. Further, in the syringe assembly, the barrel is pressed toward the distal end through the grip against the biasing force of the biasing member, so that it becomes more important to support fingers of the user by the large diameter portion of the grip so that the fingers of the user do not slip.

In the syringe assembly, the cover body may be configured so as to cover the needle tip in an initial state, be relatively displaced toward the proximal end with respect to the barrel along the outer circumferential surface of the barrel to cause the puncture needle to protrude toward the distal end by being pressed by the object to be punctured against the biasing force of the biasing member when the puncture needle is punctured into the object to be punctured, and cover the needle tip by being relatively displaced toward the distal end with respect to the barrel by the biasing force of the biasing member when the puncture needle is removed from the object to be punctured.

In a configuration in which the cover body covers the needle tip in the initial state in this way, a length where the cover body is pressed becomes longer, so that it becomes more important to support fingers by the large diameter portion of the grip so that the fingers do not slip.

In the syringe assembly, when the cover body is located at a most proximal end side in a movable range in the axis direction of the cover body with respect to the barrel, a distal end surface of the cylindrical body portion may be in contact with a proximal end surface of the cover body or located close to the proximal end surface.

By this configuration, when using the syringe assembly, the user can know how deep the puncture needle is punctured by seeing the position of the proximal end surface of the cover body while seeing the distal end surface, which is the most distal end portion of the cylindrical body portion, as a mark, so that it is possible to conduct an appropriate depth of puncture.

In the syringe assembly, the syringe may have a gasket that is slidably inserted into the barrel, a pusher that is connected to the gasket or can be connected to the gasket, and a liquid medicine filled in a liquid chamber formed by the barrel and the gasket.

According to the grip and the syringe assembly of the present invention, the grip can be easily attached to the proximal end outer circumferential portion of the barrel, and it is possible to effectively suppress slip of fingers when pressing the syringe.

### Brief Description of Drawings

Fig. 1 is a perspective view of a syringe assembly according to an embodiment of the present invention.
Fig. 2 is a perspective view of a grip according to the embodiment of the present invention.
Fig. 3A is a plan view of the grip shown in Fig. 2, and Fig. 3B is a front view of the grip shown in Fig. 2.
Fig. 4 is a perspective view of the syringe assembly during puncture.
Fig. 5 is a perspective view of the syringe assembly after use.

### Description of Embodiments

Hereinafter, a preferred embodiment of a grip and a syringe assembly according to the present invention will be described with reference to the accompanying drawings. A "user" in the description below indicates a person who administers liquid medicine (injection solution) to a patient. However, the user is not limited to a doctor, a nurse, or the like, but includes the patient himself or herself. In the description below, the "proximal end" indicates an end portion on the side close to the user who operates a syringe and the "distal end" indicates an end portion on the side away from the user.

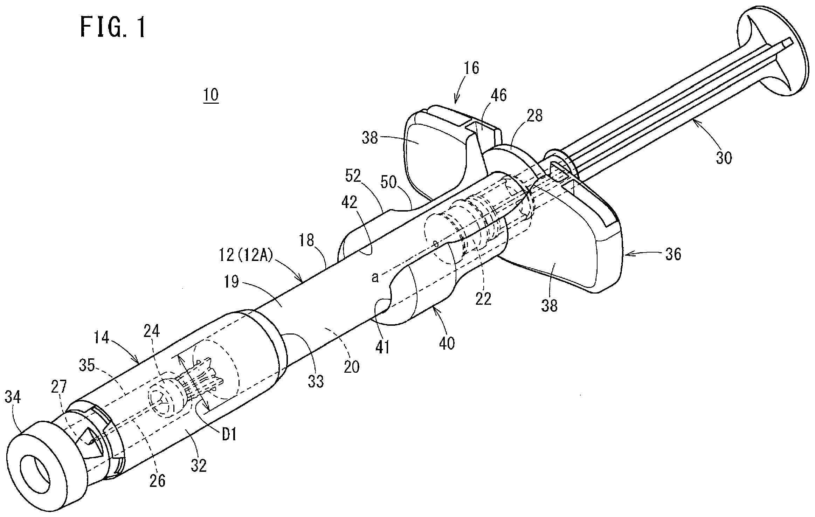

In Fig. 1, a syringe assembly 10 according to the present embodiment includes a prefilled syringe 12A as a syringe 12, a protective device 14 attached to the prefilled syringe 12A, and a grip 16 attached to the prefilled syringe 12A.

The prefilled syringe 12A includes a hollow barrel 18 that constitutes a body portion of the syringe 12, a liquid medicine 20 filled in the barrel 18, a gasket 22 inserted into the barrel 18, a needle holding portion 24 provided at the distal end of the barrel 18, and a puncture needle 26 held by the needle holding portion 24.

The barrel 18 is a hollow body that has a substantially cylindrical shape and has a proximal end opening portion at its proximal end. On a proximal end outer circumferential portion of the barrel 18, a flange 28 is formed so as to protrude toward outside in a radial direction. The gasket 22 is inserted into the barrel 18 through the proximal end opening portion. A proximal end side of the barrel 18 is liquid-tightly sealed by the gasket 22, and the liquid medicine 20 is sealed in the barrel 18.

An outer circumferential portion of the gasket 22 is liquid-tightly in contact with an inner circumferential surface of barrel 18 and is slidably arranged in the barrel 18. A distal end portion of a pusher 30 is connected to the gasket 22. When the user presses the pusher 30, the gasket 22 slides in the barrel 18. The pusher 30 may be connected to the gasket 22 when puncturing the puncture needle 26 in a patient and administering the liquid medicine 20 to the patient.

The needle holding portion 24 is integrally formed at the distal end portion of the barrel 18, extends from the distal end central portion of the barrel 18 toward the distal end while a diameter is gradually shrunk with respect to the barrel 18, and holds the proximal end side of the puncture needle 26.

The puncture needle 26 is a hollow body in which a lumen that is a fluid passage is formed. A sharp needle tip 27 is formed at the distal end of the puncture needle 26. The puncture needle 26 protrudes from the needle holding portion 24 toward the distal end. The lumen of the puncture needle 26 communicates with a hollow portion (liquid chamber) of the barrel 18 through a proximal end opening of the puncture needle 26. The puncture needle 26 is fixed to and held by a center portion of the needle holding portion 24 by an appropriate fixing method such as insert molding, thermal welding by high frequency or laser, and adhesion with adhesive. The liquid medicine 20 contained in the hollow portion of the barrel 18 is discharged from the distal end of the lumen of the puncture needle 26 and administered to the patient.

The protective device 14 is a device to prevent careless puncture to the patient other than puncture for administering the liquid medicine 20 to the patient by covering the puncture needle 26 before performing puncture of the puncture needle 26 into the patient and after performing the puncture. The protective device 14 includes at least a hollow cylindrical cover body 32 relatively displaceable in an axis direction with respect to the barrel 18.

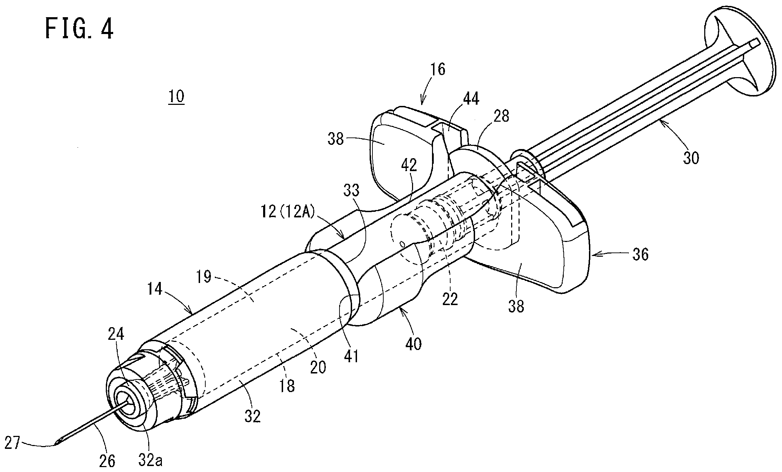

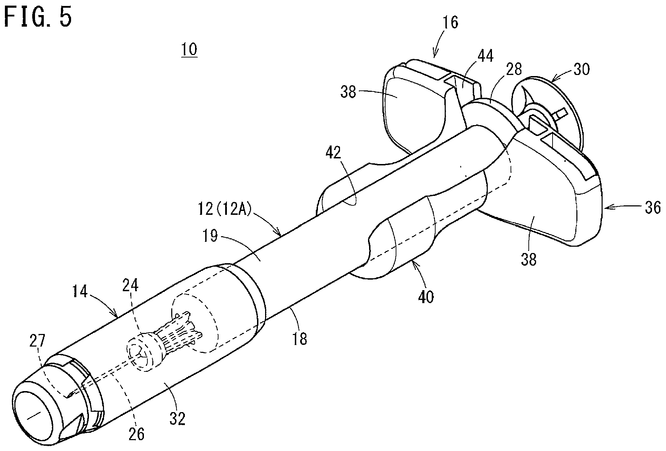

The cover body 32 is configured so as to cover the needle tip 27 of the puncture needle 26 in an initial state before use (see Fig. 1), be relatively displaced toward the proximal end with respect to the barrel 18 to cause the puncture needle 26 to protrude from the distal end of the cover body 32 toward the distal end when puncturing the puncture needle 26 to the patient who is an object to be punctured (see Fig. 4), and be relatively displaced toward the distal end with respect to the barrel 18 after the puncturing to cover the needle tip 27 of the puncture needle 26 again (see Fig. 5).

A configuration of the protective device 14 including the cover body 32 functioning in this way is not limited to a specific configuration but may have various forms. Therefore, the protective device 14 maybe configured, for example, as described below or may be configured to other forms.

The protective device 14 may include an inner member rotatably attached to an outer circumference of the needle holding portion 24, the cover body 32 relatively movable in the axis direction with respect to the inner member, and a spring member (biasing member) that biases the cover body 32 toward the distal end. Inthiscase, a camprotrusionprotruding outward in the radial direction is formed on the outer circumferential portion of the inner member. The cover body 32 is composed of a hollow body having a side wall forming a through hole extending from the distal end to the proximal end and the inner member is housed in the through hole. A guide path is formed in the side wall of the cover body 32 and the cam protrusion of the inner member is movably arranged in the guide path. The spring member is housed in the through hole of the cover body 32 and biases the cover body 32 toward the distal end with respect to the inner member.

In this case, the guide path may be configured so that the cover body 32 is relatively displaced with respect to the barrel 18 from an initial position toward the proximal end to be guided to a retreat position along with a puncture operation of the puncture needle 26 to the patient, and is relatively displaced with respect to the barrel 18 from the retreat position toward the distal end to be guided to a lock position along with a removal operation of the puncture needle 26. In this case, the cover body 32 covers the needle tip 27 of the puncture needle 26 at the initial position. The cover body 32 causes the puncture needle 26 to be protruded from a distal end opening 32a at the retreat position and covers the needle tip 27 of the puncture needle 26 at the lock position. When the cover body 32 moves from the initial position to the retreat position, the spring member is pressed in the axis direction . When the puncture needle 26 is removed, the cover body 32 is moved from the retreat position to the lock position by an elastic biasing force of the spring member. When the cover body 32 is located at the initial position, the needle tip 27 may be exposed (protruded) from the distal end opening 32a of the cover body 32.

Further, in this case, while the cover body 32 moves from the initial position to the lock position through the retreat position, the cam protrusion moves in the guide path, so that the inner member relatively rotates with respect to the cover body 32. When the cover body 32 has moved to the lock position, the inner member restricts relative displacement of the cover body 32 toward proximal end with respect to the inner member.

In Fig. 1, a cap 34 covers the puncture needle 26. The cap 34 is composed of a soft resin material such as a rubber material and has a sealed portion 35 where the puncture needle 26 is inserted. Before the syringe assembly 10 is used, the cap 34 covers the puncture needle 26 so as to seal the needle tip 27 (distal end opening of the puncture needle 26) and is inserted into the cover body 32. When using the syringe assembly 10, a user holds a distal end portion of the cap 34 and pulls out the cap 34 in the distal end direction, so that the cap 34 is removed.

Next, a configuration of the grip 16 will be described. In the present embodiment, when a product of the syringe assembly 10 is provided, as shown in Fig. 1, the grip 16 is attached to a proximal end outer circumferential portion of the prefilled syringe 12A. The grip 16 may be separated from the prefilled syringe 12A when the product is provided, and the grip 16 may be attached to the proximal end outer circumferential portion of the prefilled syringe 12A when a user uses the syringe assembly 10.

Hereinafter, regarding the grip 16, a central axial line a is simply referred to as an "axis line a" and an extending direction of the axis line a is referred to as an "axis direction" . The grip 16 includes a base portion 36 having a pair of finger rest portions 38 and a cylindrical body portion 40 protruding from the base portion 36 toward the distal end. Further, a mounting slit 42 that traverses longitudinally through a wall portion of the base portion 36 and a wall portion of the cylindrical body portion 40 in the axis direction is formed over the entire length of the grip 16.

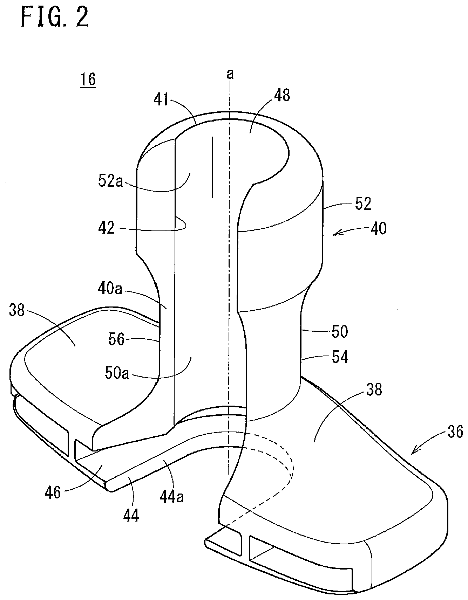

As shown in Fig. 2, the base portion 36 is a portion that forms a proximal end portion of the grip 16 and is formed into a plate shape perpendicular to the axis line a of the grip 16. The pair of finger rest portions 38 in the base portion 36 is perpendicular to the axis line a and protrude in opposite directions to each other. Thereby, the shape of the base portion 36 seen from the axis direction is formed relatively long in a protrusion direction of the finger rest portions 38 and is formed relatively short in a direction perpendicular to the protrusion direction. In a state in which the grip 16 is attached to the barrel 18, the pair of finger rest portions 38 protrudes outside of the flange 28.

A substantially U-shaped cutout portion 44 is formed in the base portion 36. The cutout portion 44 constitutes a part (proximal end region) of the mounting slit 42, penetrates in the thickness direction (axis direction) of the base portion 36, and opens in one side surface in the short length direction of the base portion 36. In an inner surface 44a of the cutout portion 44, a groove portion 46 having a substantially U-shape along the shape of the cutout portion 44 is formed as a flange mounting portion to be mounted to the flange 28. In a state in which the grip 16 is attached to the barrel 18 as shown in Fig. 1, the flange 28 of the barrel 18 is inserted into the groove portion 46, and thereby a relative movement of the grip 16 in the axis direction with respect to the barrel 18 is restricted.

The cylindrical body portion 40 protrudes from the base portion 36 toward the distal end and extends in a circumferential direction so as to fit to an outer circumferential surface 19 of the barrel 18. In other words, the cylindrical body portion 40 is formed into a shape where a hollow cylindrical shape is partially cut out in a circumferential direction as an opening portion (a cross-sectional C-shape). In Fig. 2, an opening portion 40a of the cylindrical body portion 40 constitutes a part (a distal end region and an intermediate region) of the mounting slit 42. In the cylindrical body portion 40, amounting groove 48 that penetrates in the axis direction is formed by a circumferential wall constituting the cylindrical body portion 40.

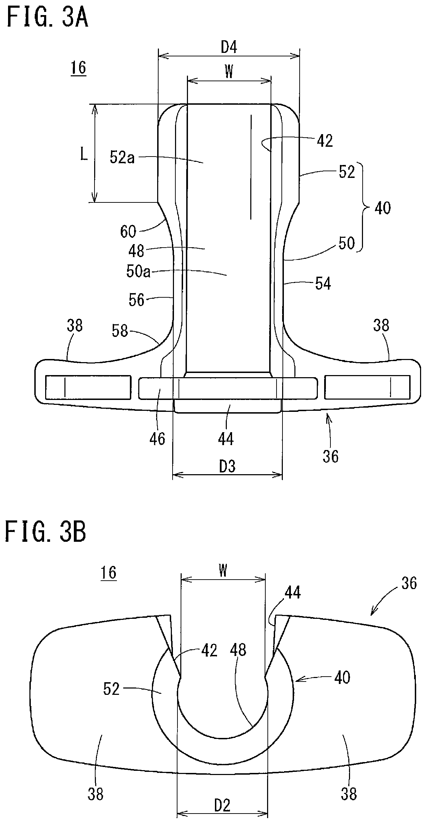

In Fig. 3B, a diameter D2 of the mounting groove 48 (an inner diameter of the cylindrical body portion 40) is constant in the axis direction over the entire length of the cylindrical body portion 40 and is set to substantially the same as an outer diameter D1 of the barrel 18 (see Fig. 1).

In Figs. 2 and 3A, the cylindrical body portion 40 has a small diameter portion 50 extending from the base portion 36 toward the distal end and a large diameter portion 52 extending from the small diameter portion 50 toward the distal end. The small diameter portion 50 constitutes an intermediate region in the axis direction of the grip 16 and is formed to be thinner than the large diameter portion 52 (an outer diameter of the small diameter portion 50 is smaller than that of the large diameter portion 52).

Although inner diameters of the large diameter portion 52 and the small diameter portion 50 are the same, a thickness of a wall portion that constitutes the large diameter portion 52 is thicker than a wall portion that constitutes the small diameter portion 50. Further, the large diameter portion 52 constitutes a distal end region of the grip 16 and is formed to be thicker than the small diameter portion 50. In other words, as shown in Fig. 3A, an outer diameter D4 of the large diameter portion 52 is greater than an outer diameter D3 of a thinnest portion of the small diameter portion 50. Therefore, a constricted portion 54 having a shape recessed inward in a radial direction is formed in an outer circumferential portion of the small diameter portion 50 due to an outer diameter difference between the large diameter portion 52 and the small diameter portion 50.

The constricted portion 54 has a straight portion 56 whose outer diameter is constant in the axis direction. The straight portion 56 constitutes a thinnest portion of the constricted portion 54. An outer circumferential surface 58 that continues to a proximal end side of the straight portion 56 spreads in a flare shape toward the proximal end and smoothly continues to a distal end surface of the finger rest portions 38. An outer circumferential surface 60 that continues to a distal end side of the straight portion 56 enlarges in diameter in a flare shape toward the distal end and continues to the large diameter portion 52.

A diameter difference between the large diameter portion 52 and the small diameter portion 50 on an outer circumferential portion of the cylindrical body portion 40 prevents fingers of a user that are hooked on the small diameter portion 50 from slipping toward the distal end when the user presses the syringe 12 toward the distal end in order to puncture a skin of a patient with the puncture needle 26 of the syringe 12. Therefore, the magnitude of the diameter difference (a difference of outer circumferential radius between an outer circumferential radius of the large diameter portion 52 and an outer circumferential radius of the thinnest portion of the small diameter portion 50 (the straight portion 56 in Fig. 3A)) is, for example, 0.5 to 5.0 mm, and is preferably 1.0 to 2.5 mm. That is to say, a difference between the outer diameter of the large diameter portion 52 and the outer diameter of the thinnest portion of the small diameter portion 50 is 1.0 to 10 mm, and is preferably 2.0 to 5.0 mm. Alternatively, a ratio of the outer diameter of the large diameter portion 52 to the outer diameter of the thinnest portion of the small diameter portion 50 is, for example, 105 to 200%, and is preferably 110 to 150%. When the difference between the outer diameter of the large diameter portion 52 and the outer diameter of the thinnest portion of the small diameter portion 50 is 5.0 mm or less or the ratio of the outer diameter of the large diameter portion 52 to the outer diameter of the thinnest portion of the small diameter portion 50 is 150% or less, a diameter difference between the barrel 18 and the large diameter portion 52 is not so large, so that it is possible to prevent a user from hooking fingers by mistake on the distal end of the large diameter portion 52. It is preferable that the distal end of the large diameter portion 52 has a shape whose diameter gradually decreases toward the distal end. Thereby, it is possible to more reliably prevent the user from hooking fingers by mistake on the distal end of the large diameter portion 52.

The mounting slit 42 is a clearance gap that penetrates an inner circumferential surface and an outer circumferential surface of the cylindrical body portion 40 in a radial direction and communicates between inside (the mounting groove 48) and outside of the cylindrical body portion 40. In Fig. 3B, an opening width W of the mounting slit 42 in a natural state where the mounting slit 42 is not elastically deformed is smaller than the inner diameter D2 of the cylindrical body portion 40 (diameter of the mounting groove 48) and the outer diameter D1 of the barrel 18 (see Fig. 1). Thereby, when the grip 16 is attached to an outer circumferential portion of the barrel 18, the mounting slit 42 is elastically deformed and pressingly expanded by the barrel 18. When the barrel 18 is inserted into the mounting groove 48, the opening width of the mounting slit 42 is returned to the original opening width by an elastic restoring force of the cylindrical body portion 40.

In the syringe assembly 10 according to the present embodiment, when the cover body 32 is located at the most proximal end side in a movable range in the axis direction of the cover body 32 with respect to the barrel 18 (when the cover body 32 has moved to the retreat position), a distal end surface 41 of the cylindrical body portion 40 (a most distal end portion of the grip 16) is in contact with a proximal end surface 33 of the cover body 32 or is located close to the proximal end surface 33 (see Fig. 4). Therefore, the distal end surface 41 of the cylindrical body portion 40 has a function of informing a user of the most proximal end position in the movable range of the cover body 32.

The large diameter portion 52 extends from the small diameter portion 50 toward the distal end while enlarging its diameter, so that not only the constricted portion 54 is formed on the outer circumference of the small diameter portion 50, but also an inner circumferential surface 52a of the large diameter portion 52 and an inner circumferential surface 50a of the small diameter portion 50 have a function to support the outer circumferential surface 19 of the barrel 18. Thereby, rattling of the grip 16 with respect to the barrel 18 is suppressed. From a viewpoint of more suitably exhibiting such a function to support the outer circumferential surface 19 of the barrel 18, a length L of the large diameter portion 52 along the axis direction (see Fig. 3A) is, for example, set to 1.5 to 20 mm, and is more preferably set to 3 to 15 mm. When the length L of the large diameter portion 52 is longer than 20 mm, a length where the grip 16 covers the barrel 18 in the axis direction is long, so that visibility of the liquid medicine filled in the barrel 18 degrades.

Constituent material of the grip 16 is not particularly limited. Examples of the constituent material include resin materials such as polypropylene, polycarbonate, polyamide, polysulfone, polyarylate, ABS, and high-density polyethylene.

Next, an operation of the syringe assembly 10 configured as described above will be described.

When administering the liquid medicine 20 to a patient, a user (may be the patient himself or herself) first connects the pusher 30 to the gasket 22 to create a state shown in Fig. 1. The syringe assembly 10 may be provided to the user in a state in which the pusher 30 is connected to the gasket 22 in advance as shown in Fig. 1.

Then a distal end portion of the cap 34 is held and pulled, so that the cap 34 is pulled out from the cover body 32 and the cap 34 is detached from the puncture needle 26. At this time point, the cover body 32 is biased to the initial position (pre-puncture position) by the elastic biasing force of the spring member described above, and a state in which the puncture needle 26 including the distal end needle tip 27 is surrounded by the cover body 32 is maintained, so that the puncture needle 26 is prevented from being carelessly punctured to the user or the like.

Next, the user holds the grip 16 by hooking two fingers (for example, the index finger and the middle finger) on the pair of finger rest portions 38 and sandwiching the small diameter portion 50 (constricted portion 54) by the two fingers. Then, the user performs simple positioning by causing a distal end portion of the cover body 32 to come into contact with a puncture position (arm or the like) of the patient and, further, presses the barrel 18 through the grip 16. By doing so, the cover body 32 is relatively displaced toward the proximal end with respect to the barrel 18 against the elastic biasing force of the spring member.

Accompanying such relative displacement, as shown in Fig. 4, the puncture needle 26 held by the needle holding portion 24 is exposed from the distal end opening 32a of the cover body 32, and the cover body 32 moves to the most proximal end position (retreat position) in the movable range with respect to the barrel 18. In this stage, the proximal end surface 33 of the cover body 32 is in contact with the distal end surface 41 of the cylindrical body portion 40 of the grip 16 or is located close to the distal end surface 41. At this time point, most of the puncture needle 26 is exposed from the distal end of the cover body 32 and is punctured into the body of the patient.

In a state in which the puncture needle 26 is punctured into the patient, the user presses the pusher 30 toward the distal end. Thereby, the liquid medicine 20 filled in the barrel 18 is discharged from the puncture needle 26 through the lumen of the puncture needle 26. As a result, the liquid medicine 20 is administered to the patient.

After the liquid medicine 20 is administered, the cover body 32 is detached from the patient along with the prefilled syringe 12A. At this time, the spring member arranged in the cover body 32 is expanded by an elastic restoring force, and the spring member biases the cover body 32 toward the distal end with respect to the barrel 18. Thereby, as shown in Fig. 5, the cover body 32 is relatively displaced toward the distal end with respect to the barrel 18, and the cover body 32 moves from the retreat position to the lock position. At a stage when the cover body 32 reaches the lock position, the puncture needle 26 including the needle tip is covered by the cover body 32, and relative displacement of the cover body 32 toward proximal end with respect to the barrel 18 is prevented. Therefore, also after the liquid medicine 20 is administered to the patient, it is possible to avoid careless puncture.

As described above, in the present embodiment, the mounting slit 42 that traverses longitudinally through the base portion 36 and the cylindrical body portion 40 in the axis direction is formed in the grip 16. Thereby, it is possible to easily attach the grip 16 to the proximal end outer circumferential portion of the barrel 18 through the mounting slit 42 from a side of the barrel 18. Further, the constricted portion 54 having a shape recessed inward in the radial direction is formed in the outer circumferential portion of the small diameter portion 50 due to the outer diameter difference between the large diameter portion 52 and the small diameter portion 50. Thereby, when the barrel 18 is pressed toward the distal end in order to puncture a skin of the patient with the puncture needle 26, it is possible to effectively prevent fingers of the user that are hooked on the constricted portion 54 from slipping toward the distal end.

In the present embodiment, the outer circumferential portion of the small diameter portion 50 has the straight portion 56 whose outer diameter is constant over a predetermined length in the axis direction. By this configuration, the user can more easily hook fingers on the constricted portion 54.

In the present embodiment, the ratio of the outer diameter D4 of the large diameter portion 52 to the outer diameter D3 of the thinnest portion of the small diameter portion 50 is 105 to 200%. By this configuration, a diameter difference of an appropriate size where the user can easily hook fingers can be formed between the large diameter portion 52 and the small diameter portion 50.

In the present embodiment, the large diameter portion 52 has the inner circumferential surface 52a whose inner diameter is constant in the axis direction and is substantially the same as the outer diameter D1 of the barrel 18 and which is configured to support the outer circumferential surface 19 of the barrel 18. By this configuration, it is possible to suppress rattling of the grip 16 in a state in which the grip 16 is attached to the barrel 18.

In the syringe assembly 10 according to the present embodiment, the protective device 14 has the cover body 32 which is relatively displaced toward the proximal end with respect to the barrel 18 along the outer circumferential surface 19 of the barrel 18 by being pressed by an object to be punctured against an biasing force of the biasing member when the puncture needle 26 is punctured into the object to be punctured and which covers the needle tip 27 by being relatively displaced toward the distal end with respect to the barrel 18 by the biasing force of the biasing member when the puncture needle 26 is removed from the object to be punctured. Further, when the cover body 32 is located at the most proximal end side in the movable range in the axis direction of the cover body 32 with respect to the barrel 18, the distal end surface 41 of the cylindrical body portion 40 is in contact with the proximal end surface 33 of the cover body 32 or is located close to the proximal end surface 33.

By this configuration, it is possible to cause the user to understand the movable range of the cover body 32 in advance before using the syringe assembly 10. Therefore, when the user uses the syringe assembly 10, it is possible to prevent the user from hooking fingers by mistake on the outer circumferential surface of the barrel 18 located closer to the distal end than the grip 16 and avoid the cover body 32 from hitting fingers of the user when the cover body 32 moves toward the proximal end during the puncture. Thereby, it is possible to prevent blocking of the movement of the cover body 32 toward the proximal end and avoid insufficient puncture that does not reach a prescribed depth. Further, in the syringe assembly 10 including the protective device 14 that biases the cover body 32 by the biasing force (spring force) of the biasing member, the barrel 18 is pressed toward the distal end through the grip 16 against the biasing force of the biasing member, so that it becomes more important to support fingers of the user by the large diameter portion 52 of the grip 16 so that the fingers of the user do not slip. Further, when using the syringe assembly 10, the user can know how deep the puncture needle 26 is punctured by seeing the position of the proximal end surface 33 of the cover body 32 while seeing the distal end surface 41, which is the most distal end portion of the cylindrical body portion 40, as a mark, so that it is possible to conduct an appropriate depth of puncture.

In particular, in the syringe assembly 10, the cover body 32 is configured so as to cover the needle tip 27 in the initial state, be relatively displaced toward the proximal end with respect to the barrel 18 along the outer circumferential surface 19 of the barrel 18 to cause the puncture needle 26 to protrude toward the distal end by being pressed by an object to be punctured against the biasing force of the biasing member when the puncture needle 26 is punctured into the object to be punctured, and cover the needle tip 27 by being relatively displaced toward the distal end with respect to the barrel 18 by the biasing force of the biasing member when the puncture needle 26 is removed from the object to be punctured. In a configuration in which the cover body 32 covers the needle tip 27 in the initial state in this way, a length where the cover body 32 is pressed becomes longer, so that it becomes more important to support fingers by the large diameter portion 52 of the grip 16 so that the fingers do not slip.

The syringe 12 is not limited to the prefilled syringe 12A but may be a syringe that is filled with the liquid medicine 20 after the syringe is provided as a product. The syringe 12 attached with the grip 16 need not be attached with the protective device 14.

The present invention is not limited to the embodiment described above but may be variously modified without departing from the scope of the present invention.

## Claims

1. A grip (16) that is attached to a barrel (18) of a syringe (12) having a flange (28) protruding outward at a proximal end portion of the barrel (18), the grip (16) comprising:
a base portion (36) having a finger rest portion (38) protruding outside of the flange (28) and a flange mounting portion to be mounted to the flange (28); and
a cylindrical body portion (40) that protrudes from the base portion (36) toward a distal end and extends in a circumferential direction so as to fit to an outer circumferential surface (19) of the barrel (18),
wherein a mounting slit (42) that traverses longitudinally through a wall portion of the base portion (36) and a wall portion of the cylindrical body portion (40) in an axis direction is formed over an entire length of the grip (16), and
wherein the cylindrical body portion (40) comprises:
a small diameter portion (50) that is relatively thin and protrudes from the base portion (36) toward the distal end, and
a large diameter portion (52) that extends from the small diameter portion (50) toward the distal end to constitute a distal end region of the grip (16) and is formed to be thicker than the small diameter portion (50).

2. The grip (16) according to claim 1, wherein
an outer circumferential portion of the small diameter portion (50) has a straight portion (56) whose outer diameter is constant over a predetermined length in the axis direction.

3. The grip (16) according to claim 1 or 2, wherein
a ratio of an outer diameter of the large diameter portion (52) to an outer diameter of a thinnest portion of the small diameter portion (50) is 105 to 200%.

4. The grip (16) according to any one of claims 1 to 3, wherein
a difference between an outer diameter of the large diameter portion (52) and an outer diameter of a thinnest portion of the small diameter portion (50) is 1.0 to 10 mm.

5. The grip (16) according to any one of claims 1 to 4, wherein
the large diameter portion (52) has an inner circumferential surface (52a) whose inner diameter is constant in the axis direction and which is configured to support the outer circumferential portion of the barrel (18).

6. The grip (16) according to claim 5, wherein
a length of the large diameter portion (52) along the axis direction is 1.5 to 20 mm.

7. A syringe assembly (10) comprising:
a syringe (12) having a barrel (18); and
a grip (16) attached to a proximal end outer circumferential portion of the barrel (18),
wherein the grip (16) is the grip (16) according to any one of claims 1 to 6.

8. The syringe assembly (10) according to claim 7, further comprising:
a protective device (14) attached to the syringe (12),
wherein the syringe (12) has a puncture needle (26) having a needle tip (27) and the barrel (18) that holds the puncture needle (26),
the protective device (14) has a hollow cylindrical cover body (32) that is relatively displaceable with respect to the barrel (18) in an axis direction and an biasing member that biases the cover body (32) toward a distal end with respect to the barrel (18), and
the cover body (32) is configured to be relatively displaced toward a proximal end with respect to the barrel (18) along an outer circumferential surface (19) of the barrel (18) by being pressed by an object to be punctured against an biasing force of the biasing member when the puncture needle (26) is punctured into the object to be punctured, and cover the needle tip (27) by being relatively displaced toward the distal end with respect to the barrel (18) by the biasing force of the biasing member when the puncture needle (26) is removed from the object to be punctured.

9. The syringe assembly (10) according to claim 8, wherein
the cover body (32) is configured so as to cover the needle tip (27) in an initial state, be relatively displaced toward the proximal end with respect to the barrel (18) along the outer circumferential surface (19) of the barrel (18) to cause the puncture needle (26) to protrude toward the distal end by being pressed by the object to be punctured against the biasing force of the biasing member when the puncture needle (26) is punctured into the object to be punctured, and cover the needle tip (27) by being relatively displaced toward the distal end with respect to the barrel (18) by the biasing force of the biasing member when the puncture needle (26) is removed from the object to be punctured.

10. The syringe assembly (10) according to claim 8 or 9, wherein
when the cover body (32) is located at a most proximal end side in a movable range in the axis direction of the cover body (32) with respect to the barrel (18), a distal end surface of the cylindrical body portion (40) is in contact with a proximal end surface of the cover body (32) or is located close to the proximal end surface.

11. The syringe assembly (10) according to any one of claims 7 to 10, wherein
the syringe (12) has a gasket (22) that is slidably inserted into the barrel (18), a pusher (30) that is connected to the gasket (22) or can be connected to the gasket (22), and a liquid medicine (20) filled in a liquid chamber formed by the barrel (18) and the gasket (22).
